# EUROPEAN PATENT APPLICATION

(11) **EP 2 921 053 A1**
(43) Date of publication of application: **23.09.2015**
(21) Application number: 14305401.3
(22) Date of filing: 21.03.2014
(51) Int. Cl.: A01N 65/12, A61K 36/28, A01P 3/00

(54) **Extracts of Santolina chamaecyparissus**

(71) Applicant: Naturex, 84140 Avignon (FR); Société Française d'ingénierie appliquée aux cosmétiques, 06510 Carros (FR); Centre National de la Recherche Scientifique (CNRS), 75794 Paris Cedex 16 (FR); Agence Conseil pour la Phytothérapie et l'Aromathérapie, 06530 Cabris (FR); Université De Nice Sophia Antipolis, 06103 Nice (FR)
(72) Inventor: Fernandez, Xavier, 06100 Nice (FR); Merck, Florence, 67630 Lauterbourg (FR); Monin, Claude, 06530 Cabris (FR); Rolland, Yohan, 84310 Morières les Avignon (FR); Kerdudo, Audrey, 06200 Nice (FR); Dingas, Alexandre, 06110 Le Cannet (FR)
(74) Representative: Cabinet Plasseraud

(57) **Abstract**

The invention relates to an extract of *Santolina chamaecyparissus,* to a process for preparing said extract, to a composition comprising it, and to its use a preservative, particularly as antioxidant, bactericidal and/or fungicidal.

## Description

The invention relates to a novel extract of *Santolina chamaecyparissus L.,* to a composition comprising it, and to cosmetic and dermatological methods using same, especially as preservative, bactericide, fungicide and/or antioxidant.

Topical cosmetic, toiletry and pharmaceutical products such as creams, lotions, pastes, liquids, aerosols, shampoos, gels, wipes, bats, sticks, powders and granules, are known in the art to be susceptible to microbial infestation. The raw materials, packaging, and manufacturing environment for these products are often not sufficiently sterile, such that small amounts of microbiological contaminants can enter into final products. Often, a cosmetic, toiletry or pharmaceutical product may be exposed to higher temperatures than recommended which can also accelerate the growth rate of microbes unless a suitably effective antimicrobial component and/or components are incorporated into the formulation. Once product packages are opened, they are subject to further contamination from repeated consumer use. Microbial growth can cause discoloration and/or unpleasant odor of the product, but can also degrade chemical and/or active compounds in the cosmetic, toiletry or pharmaceutical formulation, which can lead to instability of the product and/or emulsion. A product that has been contaminated by microbiological organisms can also lead to user infections once it is applied to the skin, scalp and/or mucous membranes of a human.
It is therefore important for manufacturers and marketers of such products to be able to offer products that resist microbial growth and provide a stable and safe product with a long shelf life.

Preservatives help maintaining the integrity of cosmetic, toiletry or pharmaceutical formulations. A preservative corresponds to any substance or mixture of substances able to prevent chemical or microbiological degradation of a product. More specifically, antimicrobial preservatives prevent bacterial or fungal damage (microscopic fungi or yeast). A preservative also prevents oxidation of a product, which may be related to its composition or its environment. An optimum preservative thus includes both efficacious antimicrobial and antioxidant effects. The preservatives widely used in the cosmetic industry include chemical compounds like parabens, imidazolidinyl urea, DMDM Hydantoin or phenoxyethanol. However environmental and health concerns prevail regarding the usage of such chemical preservatives. For example, parabens are becoming increasingly controversial, particularly because of their potential estrogen-mimicking aspect (Golden R, Gandy J, Vollmer G (2005). "A review of the endocrine activity of parabens and implications for potential risks to human health". Critical Reviews in Toxicology 35 (5): 435-58, Darbre et al (2004) "Concentrations of parabens in human breast tumours", J.Appl.Toxicol. 24, 5-13).

There is thus an impetus to develop natural alternatives to chemical preservatives. As natural ingredients appeal to consumers and as plants are known to possess biological activities, there is thus a need for a natural antimicrobial ingredient to replace synthetic ones.

Surprisingly, the inventors discovered and developed preservatives coming from vegetal source, which include ingredients that have both antimicrobial and antioxidant effects.
The present inventors have hence sought to develop a natural plant material based preservative that dually functions as a sustained antimicrobial and antioxidant system and thus would enhance the shelf life of cosmetic and personal care formulations.

According to a first aspect, the invention therefore relates to an extract of *Santolina chamaecyparissus L.* obtained by extraction of the aerial parts with a solvent chosen from methanol, dichloromethane, ethyl acetate, acetone, tetrahydrofuran and a mixture ethanol : water in a ratio of from 70:30 % v/v to 80:20 % v/v.

Specifically, the invention relates to a process for preparing an extract of *Santolina chamaecyparissus L*., comprising the following steps:
a) mixing aerial parts of *Santolina chamaecyparissus L.* with a solvent chosen from methanol, dichloromethane, ethyl acetate, acetone, tetrahydrofuran and a mixture ethanol : water in a ratio of from 70:30 % v/v to 80:20 % v/v,
b) macerating the mixture obtained in a) during at least 1h, preferably at ambient temperature,
c) filtering the mixture obtained in b), so as to obtain the filtrate and the retentate, the filtrate being the extract.

The invention also relates to an extract of *Santolina chamaecyparissus L.* obtainable by said process, and to compositions comprising it.

Said extract or composition may be used as preservative, antioxidant, bactericide and/or fungicide.

Finally, the invention also relates to the use of the compound of formula (I): 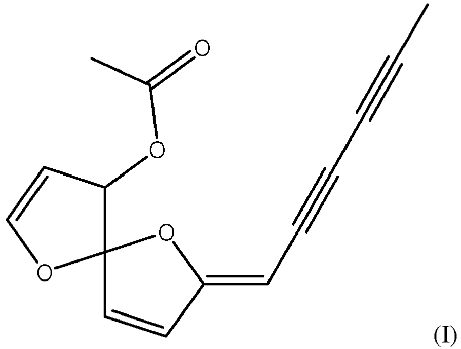 as preservative, antioxidant, bactericide and/or fungicide. Said compound is comprised in the extract according to the invention. Its chemical name is 1,6-dioxaspiro[4,4]nona-2,8-dien-4-ol,7-(2,4-hexadiyn-1-ylidene)-,4-acetate.

Particularly, in view of the above, and of the present description, said extract according to the invention is not an essential oil. Indeed, it is not obtained by distillation, but by extraction; it has thus a different composition.

In the present invention, "preservative" means any substance or mixture of substances which prevents chemical and microbiological degradation of a product comprising it. Particularly, a preservative is a substance or mixture of substances which prevents degradation by bacteria, fungi and yeast, of a product comprising it.

Typically, the preservative properties of a substance or mixture of substances are evaluated on different strains of bacteria, like gram-positive bacteria and gram-negative bacteria; on yeasts; and on fungi. Typical gram-positive bacteria are *Pseudomonas aeruginosa*; typical gram-negative bacteria are *Staphylococcus aureus* or *Escherichia coli*; typical yeasts are *Candida albicans*; and typical fungi are *Aspergillus brasiliensis* or *Aspergillus niger.*
Thus, a preservative is a bactericide, a fungicide and an antioxidant.
"Antimicrobial" means bactericide and/or fungicide.
"Bactericide" means any substance or mixture of substances which prevents degradation by bacteria of a product comprising it.
In the same way, "fungicide" means any substance or mixture of substances which prevents degradation by fungi of a product comprising it.
"Antioxidant" means any substance or mixture of substances which prevents oxidation of a product comprising it.

The extract of *Santolina chamaecyparissus L.* according to the invention is obtained by extraction of the aerial parts with a solvent chosen from methanol, dichloromethane, ethyl acetate, acetone, tetrahydrofuran and a mixture ethanol : water in a ratio of from 70:30 % v/v to 80:20 % v/v. Preferably, the extract comprises the compound of formula (I) mentioned above.
*Santolina chamaecyparissus* is a plant belonging to the family Asteraceae. Their flowers are yellow. The aerial parts of the plant may be flowers, stems, seeds, fruits or leaves. Preferably, said aerial parts comprise at least flowers.

The solvent used for extraction is very important in the present invention: indeed, it ensures the good properties of the final extract used as preservative.
Said solvent is chosen from:
- methanol,
- dichloromethane,
- ethyl acetate,
- acetone,
- tetrahydrofuran and
- a mixture ethanol : water in a ratio of from 70:30 % v/v to 80:20 % v/v.
If methanol is used, it is pure in the present invention (i.e. the solvent is 100% methanol). As shown in example 5, methanol, dichloromethane, ethyl acetate, acetone or tetrahydrofuran ensure a high concentration of the compound of formula (I) in the extract, and said compound is in part responsible for the good preservative and antioxidant activities (see example 4).
If a mixture ethanol : water is used, then ethanol and water have to be in a ratio of from 70:30% v/v to 80:20% v/v. Preferably, said ratio is of from 75:25 % v/v to 80:20 % v/v. For example, if one would like to use a ratio 75:25% v/v, then 75ml of ethanol have to be used in mixture with 25ml of water so as to obtain 100ml of solvent. As shown in examples 2 and 5, said ratio ensures a high concentration of the compound of formula (I) in the extract, and said compound is in part responsible for the good preservative and antioxidant activities (see example 4). Indeed, as shown in example 2, the antimicrobial activity with said mixture is as good as the one obtained with methylparaben.

The invention also relates to a process for preparing an extract of *Santolina chamaecyparissus L*., comprising the following steps:
a) mixing aerial parts of *Santolina chamaecyparissus L.* with a solvent chosen from methanol, dichloromethane, ethyl acetate, acetone, tetrahydrofuran and a mixture ethanol : water in a ratio of from 70:30 % v/v to 80:20 % v/v,
b) macerating the mixture obtained in a) during at least 1h, preferably at ambient temperature,
c) filtering the mixture obtained in b), so as to obtain the filtrate and the retentate, the filtrate being the extract.

Step a) of the process comprises mixing said aerial parts with a solvent chosen from methanol, dichloromethane, ethyl acetate, acetone, tetrahydrofuran and a mixture ethanol : water in a ratio of from 70:30 % v/v to 80:20 % v/v. Preferably, if a mixture ethanol:water is used, the ratio of said mixture ethanol : water is from 75:25 % v/v to 80:20 % v/v, most preferably it is either around 75:25% v/v or around 80:20 % v/v. The aerial parts may first be dried and ground into powder, before mixing with the solvent. Typically, the aerial parts of the plant are mixed with the required solvent in a ratio of 1 for 10 (i.e. 100g of aerial parts of the plant for 1L of solvent).

Then, the process comprises a step b) of macerating the mixture obtained in step a) during at least 1h. Preferably, step b) is performed at ambient temperature. By "ambient temperature", it is meant a temperature between 20 and 25°C, preferably around 25°C. Preferably, step b) lasts at least 2h. Preferably, step b) is performed under mechanical stirring.

Thereafter, the mixture obtained in step b) is filtered in step c). The solution is indeed filtered in order to remove the insoluble substances. This filtration step is usual in the plant extract field, and those skilled in the art are able to adjust the reaction parameters thereof, on the basis of their general knowledge.
At the end of step c), a filtrate and a retentate are obtained. The filtrate corresponds to an extract of the invention.

Steps a) to c) of the process according to the invention correspond to an extraction.

The process according to the invention may also comprise a further (second) extraction. More specifically, said process may preferably comprise a further step d) comprising:
d1) mixing the retentate of step c) with the same solvent as the one used in step a),
d2) macerating the mixture obtained in d1) during at least 1h, preferably at ambient temperature,
d3) filtering the mixture obtained in d2), so as to obtain the filtrate and the retentate, and
d4) mixing the filtrate of step c) with the filtrate of step d3), said final mixture being the extract.

The features of step a) described above are also applicable to step d1).
The features of step b) described above are also applicable to step d2).

The features of step c) described above are also applicable to step d3).
Finally, the last step d4) comprises mixing both filtrates of each extraction, so as to obtain a final filtrate, corresponding to an extract according to the invention.

The process according to the invention may also comprise a further step e) of evaporation of the solvent of the extract. Said evaporation may occur according to classical methods known in the art, like vacuum-concentration.
Preferably, step e) comprises addition of a liquid cosmetic compound, and evaporation of the solvent (i.e. methanol or the mixture ethanol:water in the required ratio). Said step e) may be called a step of solvent substitution. Preferably, step e) comprises:
e1) the addition of a cosmetic compound chosen from polyols and monoglycerides to the extract, and
e2) the evaporation of the solvent of the extract obtained in e1), such as vacuum-concentration.
Step e1) is typically performed at ambient temperature. Step e2) is typically performed by heating at a temperature comprised between 50°C and 70°C, typically at around 60°C. If the polyol or the monoglyceride which is used is solid at ambient temperature, then said polyol or monoglyceride is previously heated so as to become liquid or semi-liquid at ambient temperature, before step e1).
The cosmetic compound chosen from polyols and monoglycerides is cosmetically acceptable, and is liquid or semi-liquid. By "cosmetically acceptable", it is meant a compound compatible with the skin and its appendages. By "liquid or semi-liquid", it is meant liquid or pasty. Typically, a liquid or semi-liquid compound is easy to mix in a cosmetic preparation at 30°C.
The polyols are also called glycols: they correspond to organic compounds comprising at least two -OH groups. Preferably, the polyols are diols (ie they comprise 2 -OH groups), like substituted or non-substituted 1,2-propanediols, ethylene glycol, diethylene glycol, propylene glycol; or they are triols (ie they comprise 3 -OH groups), like 1,2-octanediol or glycerol.
The monoglycerides comprise glycerol monolaurate, glycerol monocaprate, glycerol monocaprylate, glycerol monooleate, glycerol monomyristate, glycerol monopalmitate and glycerol monostearate.
Preferably, the cosmetic compound is chosen from diols, triols and monoglycerides. Among the substituted 1,2-propanediols, ethylhexylglycerin is preferred. 1,2-octanediol is also called caprylyl glycol.
More preferably, the cosmetic compound is chosen from ethylhexylglycerin, ethylene glycol, diethylene glycol, propylene glycol, 1,2-octanediol (or caprylyl glycol) and glycerol.
More preferably, the cosmetic compound is chosen from ethylhexylglycerin, caprylyl glycol and their mixtures. A preferred mixture is the mixture ethylhexylglycerin/caprylyl glycol sold under the name Sensiva SC10 by Schülke & Mayr.
In this case, the extract of *Santolina chamaecyparissus L.* which is obtained at the end of step e2) comprises the cosmetic compound, preferably ethylhexylglycerin or caprylyl glycol, as solvent.

The present invention also relates to an extract of *Santolina chamaecyparissus L.* obtainable by the process described above. Said extract preferably comprises at least the compound of formula (I):

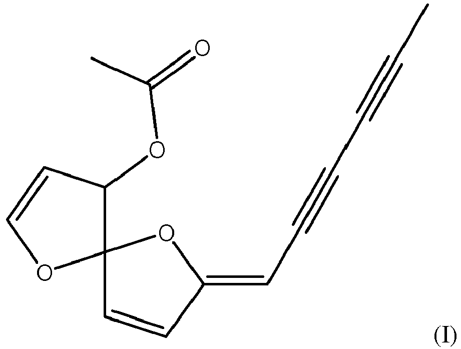

The present invention also relates to a composition, particularly cosmetic or dermatological, comprising, in a physiologically acceptable medium, an extract of *Santolina chamaecyparissus L.* according to the invention. By "physiologically acceptable medium", it is meant a medium compatible with the administration to a subject. Preferably, the composition comprises from 0.1% to 10% by weight of the extract of *Santolina chamaecyparissus L.* according to the invention, more preferably from 0.2% to 7%, more preferably from 0.3% to 5% by weight, more preferably from 0.3% to 1% by weight.

Said composition may be cosmetic, pharmaceutical (preferably dermatological), a food composition, or a chemical composition.
Said cosmetic or pharmaceutical (preferably dermatological) composition can in particular be suitable for topical application.
Advantageously, said cosmetic or pharmaceutical (preferably dermatological composition) can be in the form of a powder, an emulsion, a microemulsion, a nanoemulsion, a suspension, a solution, a lotion, a cream, an aqueous or aqueous-alcoholic gel, a foam, a serum, an aerosol solution or dispersion, or a dispersion of lipid vesicles.
In the case of an emulsion, it may be a water-in-oil or oil-in-water emulsion.
The cosmetic or pharmaceutical (preferably dermatological) composition according to the invention also comprises a solvent chosen according to the various ingredients and to the administration form.
By way of examples, mention may be made of water (preferably demineralized water) or an alcohol such as ethanol.
Said cosmetic or pharmaceutical composition can also comprise at least one additive that is usual in the field, such as, for example, at least one compound chosen from an emollient or humectant, a gelling agent and/or thickener, a surfactant, an oil, an active agent, a dye, an active agent, an organic or inorganic powder, pigments, a sunscreen and a fragrance.
In particular, said composition can comprise:
- one or more emollient(s) or humectant(s), which can be chosen, for example, from glycerine and other glycols. Said emollient or humectant may be present in the composition at a content of the order of 0.1% to 30%, preferably 2% to 10% by total weight of the composition;
- one or more aqueous phase gelling agent(s) and/or thickener(s), chosen, for example, from cellulose derivatives, gums of plant origin (guar, carob, alginates, carrageenans, pectin) or of microbial origin (xanthan), clays (laponite), acrylate copolymers, like acrylates/C10-30 alkyl acrylate crosspolymer. Said gelling agent and/or thickener may be present in the composition at a content of the order of 0.1% to 10% by total weight of the composition;

- one or more surfactant(s), preferably non-ionic, present at a content of the order of 0.1% to 10%, preferably 0.5% to 8% by total weight of the composition;
- one or more fatty substance(s) that is (are) liquid at ambient temperature, commonly called oil(s), that may be volatile or non-volatile, hydrocarbon-based or silicone-based, linear, cyclic or branched, for example vegetal oils (jojoba oil, weat germ oil), isododecane, octyldodecanol, squalane, isohexadecane or dimethicone, preferably in a proportion of 0.1% to approximately 20%, preferably 0.5% to 15% by total weight of the composition;
- one or more active agent(s) of natural or synthetic origin having a biological activity, for example chosen from vitamins, allantoin or plant extracts; and/or
- one or more water-soluble dye(s), preferably in a proportion of 0.1% to approximately 2% by total weight of the composition.
Other additives normally used in cosmetics or in pharmacy can also be present in the composition according to the invention, in particular fragrances well known in the technical field.
Those skilled in the art are capable of choosing, among all these possible additives, both the type and the amount of those which will be added to the composition, in such a way that the latter conserves all its properties.

The invention also relates to the use of an extract of *Santolina chamaecyparissus L.* according to the invention, or to the use of a composition comprising it, as preservative, antioxidant, bactericide and/or fungicide.

The invention also relates to the use of the compound of formula (I): 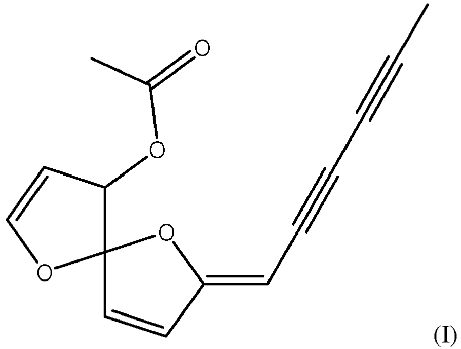 as preservative, antioxidant, bactericide and/or fungicide.

The invention also relates to the use of a composition comprising the compound of formula (I): 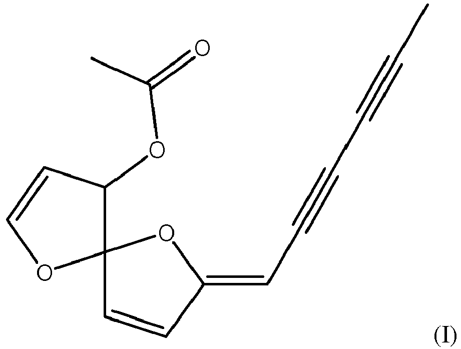 as preservative, antioxidant, bactericide and/or fungicide.

The invention is illustrated in a non limiting manner by the examples below.

### Example 1: Plant extraction of Santolina chamaecyparissus L.

Aerial parts of *Santolina chamaecyparissus* L. (also called "santolina" in the examples, unless indicated otherwise) were harvested, dried, ground into powder and extracted under mechanical stirring using a mixture of water and ethanol (75:25 v/v) (steps a) and b)). The extraction was repeated once (step d)), and both extractions were performed at 25°C during 2 hours (step c)). The obtained extracts were filtered, stirred together and vacuum-concentrated (step e)).

Crude extracts were analyzed by HPLC using an Agilent 1200 system equipped with a DAD and an ELSD detectors. Separation was performed using a Luna C18 column (Phenomenex, 250 x 4.6 mm; 5 µm). HPLC grade acetonitrile, water and 2-propanol (Sigma-Aldrich), acidified with formic acid 0.1%, were used according to the following gradient (Table 1):

**Table 1 : Solvent gradient used for HPLC-ELSD analysis**

| Time (min) | Water (%) | Acetonitrile (%) | 2-propanol (%) |
|---|---|---|---|
| 0-5 | **95** | **5** | **-** |
| 5-35 | **0** | **100** | **-** |
| 35-45 | **0** | **100** | **-** |
| 45-50 | **-** | **40** | **60** |
| 50-60 | **-** | **40** | **60** |
| 60-65 | **5** | **95** | **-** |
| 65-70 | **5** | **95** | **-** |

The characteristic analytical profile (HPLC-ELSD) of *S. chamaecyparissus* was obtained (data not shown).

### Example 2: Evaluation of the antimicrobial and antioxidant activities of the crude extract obtained in example 1

The crude extracts mentioned in this example were obtained in example 1.

### 1) Antimicrobial activity assay

### Background and samples preparation:

Antimicrobial activity of the crude extracts was determined using a 96-well microtiter plate assay based on growth inhibition. The assay was performed on four different microbial strains, chosen according to the European Pharmacopoeia (ATCC references): *Staphylococcus aureus* (gram-negative bacteria), *Pseudomonas aeruginosa* (gram-positive bacteria), *Aspergillus niger* (fungus) and *Candida albicans* (yeast).

Samples for antimicrobial assay were prepared as follows: crude extracts were diluted at 200 mg/mL in a mixture of appropriate solvents, e.g. ethanol and water (60:40 v/v). After solubilization, the solutions were filtrated on 0.45 µm syringe filters.

### Assay procedure:

The assay was performed for each strain according as follows: samples (at 200 mg/mL) were first diluted to 4 % in water, then to 2 % and 0.2 % in wells. Final concentrations were also 0.4 and 0.04 % of crude extracts in wells. Each concentration was assessed in replicate with one supplementary control well containing no microbial strain. For the assay at 2 %, samples were mixed up in the wells with growth medium (95 µL) and microbial suspension (representing an absorbance of 0.6 for *C. albicans, S. aureus* and *P. aeruginosa* and 40 spores/µL for *A. niger*). For the assay at 0.2 %, samples were first diluted to 0.2 % with water in the wells. Negative controls constituted of the solvent mixture used to prepare the sample solutions, and positive controls with a synthetic preservative (methylparaben, positive standard) were also prepared. Results were also compared with those obtained using a commercial natural preservative (commercial preservative). The 96-well plates were incubated at 25°C.

The absorbance was read for each plate at 620 nm 24, 48 and 72 hours after the beginning of the incubation to evaluate the growth of each microorganism in presence of crude extracts.

The results were expressed as a percentage of growth inhibition by the samples for each microorganism.

### 2) Results for S. chamaecyparissus crude extracts

*S. chamaecyparissus* crude extracts exhibited antimicrobial activity as shown in Table 2 (the results presented correspond to one reference crude extract, which is representative of most of extracts that have been evaluated):

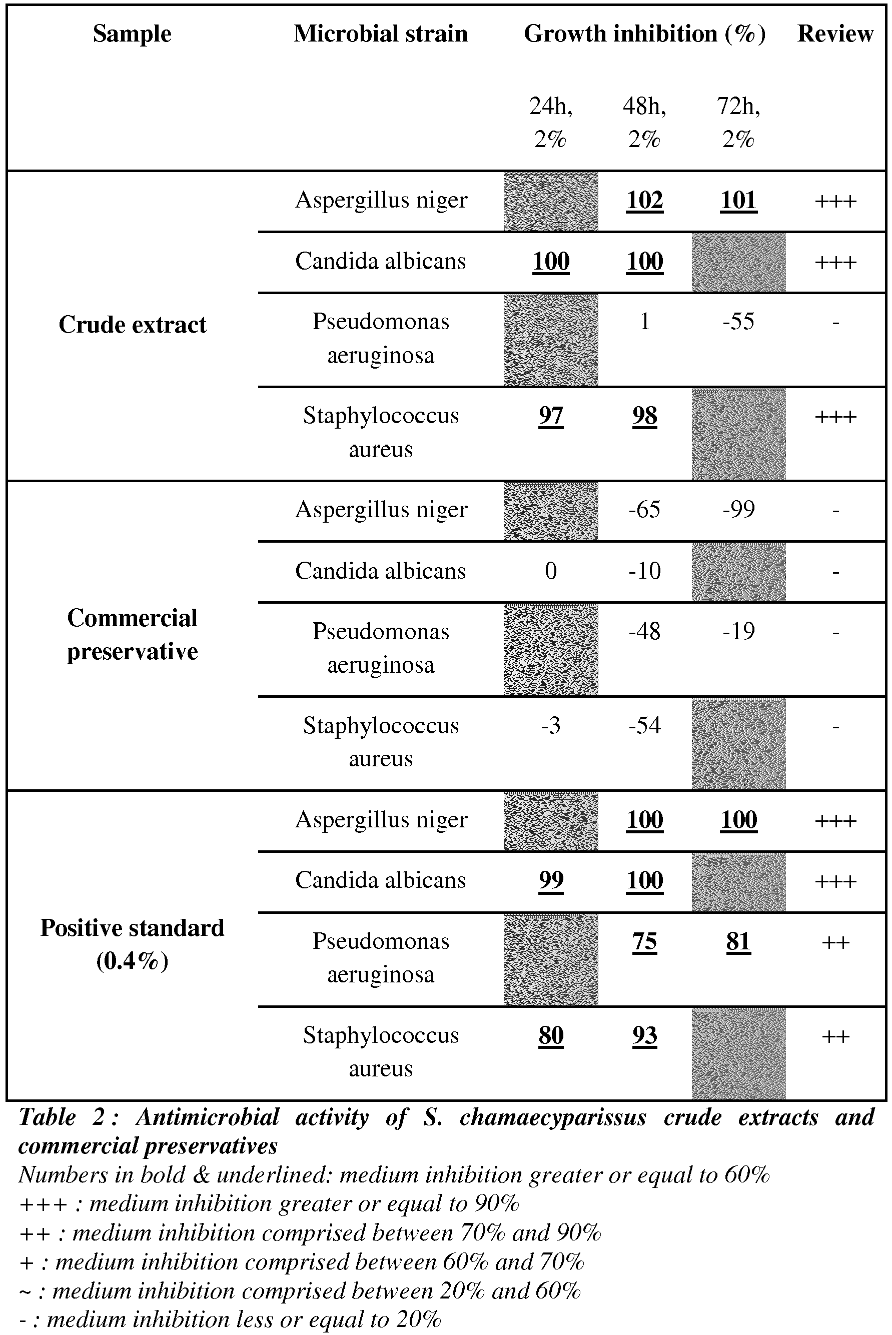

### Positive standard: methylparaben

=> As antimicrobial activity was considered to be interesting above 60 % of growth inhibition, *Santolina chamaecyparissus* crude extracts showed a very interesting activity on 3 of the 4 microbial strains, e.g. *S. aureus, A. niger* and *C. albicans.* It was interesting to notice that a commercial extract sold for its preservative properties did not show any activity, in contrast to the positive standard (methylparaben).

### 3) Description of the antioxidant assays

Sample solutions for antioxidant assays were prepared in methanol, sonicated and filtered on 0.45 µm syringe filters.

### Total phenolic content (Folin-Ciocalteu assay)

Total phenolic content of santolina crude extracts was determined using the Folin-Ciocalteu method based on oxidation of a phenolate ion from an antioxidant sample and reduction of the phosphotungstic-phosphomolybdate reagent (Folin-Ciocalteu reagent) under alkaline conditions. As a result of this reduction, the chromophore moiety turns into from a yellow complex to a blue phosphotungstic-phosphomolybdic, representative of the presence of phenols (antioxidant biological species) in the sample. The concentration of these biological species is proportional to the light absorption at a wavelength of 750 nm, where other biological species do not absorb. The absorbance values of the samples were compared to a standard, which is commonly gallic acid. Total phenolic content is thus expressed as gallic acid equivalent (GAE) in milligrams per gram of plant extract.

### Antiradical power: DPPH assay

The antiradical power of the sample was measured by the DPPH (2,2-diphenyl-1-picryl-hydrazyl) electron-transfer assay. DPPH is a free radical, which is purple in solution and reduced and getting yellow to colorless in presence of an antioxidant sample. The antiradical power of a sample is thus measured by the capacity to neutralize free radicals responsible for oxidation. DPPH reduction is observed by a spectrophotometric measurement of absorbance value at 520 nm (the lower the absorbance, the stronger the antioxidant activity). The IC₅₀ (inhibiting concentration 50%) is the parameter determined with this assay. It corresponds to the concentration of antioxidant sample that reduces 50% of the initial DPPH concentration, expressed in micrograms of sample per milliliters of methanol.

### 4) Antioxidant activity of crude extracts

Different *S. chamaecyparissus* crude extracts were evaluated (different origins and harvesting periods) and showed a total phenolic content (GAE) varying between 33 and 70 mg/g. As standards, natural rosemary extracts commercialized for their high antioxidant properties were evaluated and expressed a total phenolic content of about 85 mg/g. Another commercial antioxidant extract made of wasabi was evaluated and expressed only about 27 mg/g GAE.

Concerning the DPPH assay, the lowest IC₅₀ of *S. chamaecyparissus* extracts was 118 µg/mL. As a comparison, an extract of rosemary, known for its antioxidant potential, presented a very similar IC₅₀, namely 103 µg/mL.

=> As a conclusion, the *S. chamaecyparissus* crude extracts according to the invention seem to present an interesting antioxidant potential.

### Example 3: Evaluation of the antimicrobial and antioxidant activity of the fractions isolated from the crude extract of example 1

### 1) Fractionation of the crude extract

As the reference *S. chamaecyparissus* extract (ethanol:water 75:25 v/v) exhibited a strong antimicrobial activity, bioguided fractionation was performed to identify the fraction(s) containing active metabolites. Open column chromatographic fractionation of the crude extract was performed.

Fractionation was performed on a silica gel (normal phase) open column and yielded five fractions: cyclohexane fraction (F1), cyclohexane:ethyl acetate fraction (F2), ethyl acetate fraction (F3), ethyl acetate:methanol fraction (F4) and methanol fraction (F5).

All obtained fractions were analyzed by HPLC-ELSD in the same conditions as the crude extracts to have a qualitative idea of the different metabolites constituting each fraction.

### 2) Antimicrobial activity of santolina fractions

Fractions obtained from *S. chamaecyparissus* crude extract were submitted to antimicrobial activity assays (as explained in example 2) if sufficient quantity was present. Sample solutions were prepared at 100 mg/mL in DMSO and assessed the same way as crude extracts.

The results were as following in Table 3 (only fractions with an activity, even weak, are shown):

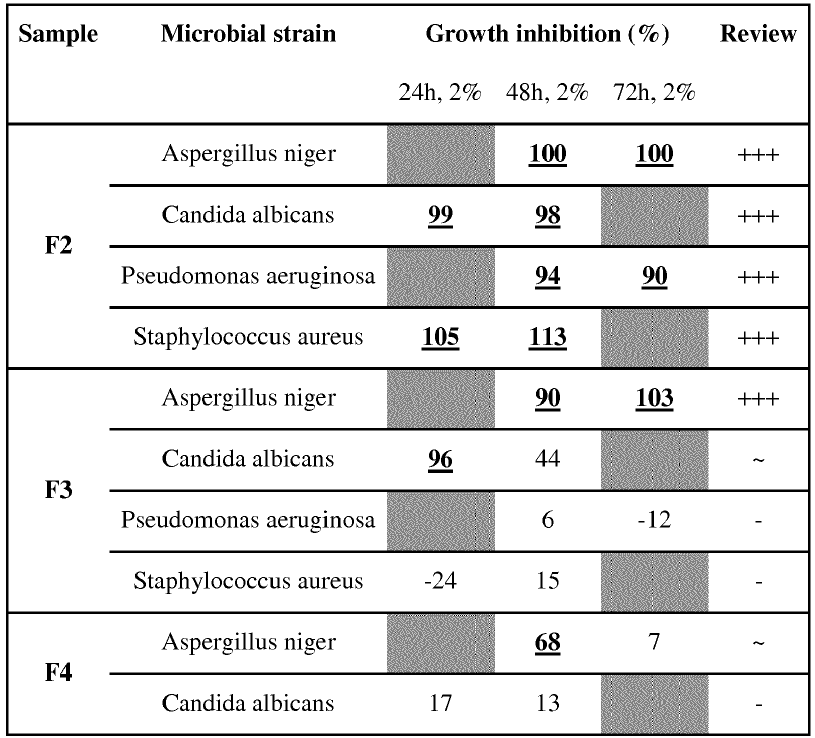

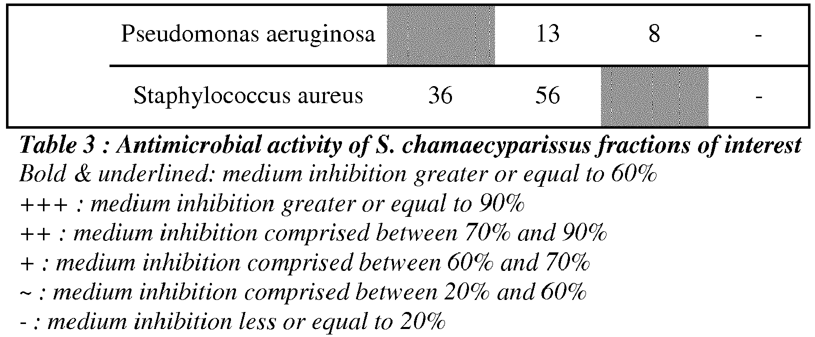

=> F2 cyclohexane:ethyl acetate fraction showed an increased activity. The presence of one major metabolite in this fraction that is not present in the other normal-phase fractions, is noticed, and this metabolite could be responsible for the antimicrobial activity (or part of) of santolina crude extracts.

### Example 4: Isolation of the active metabolite and properties thereof

Semi-preparative HPLC was performed on the active cyclohexane:ethyl acetate santolina fraction in order to purify the major metabolite supposed to be responsible for the antimicrobial activity. It was carried out on a Luna C18 column (Phenomenex, 250x10 mm; 5 µm). Elution profile was optimized and the single component of interest was successfully isolated using a gradient of non-acidified HPLC grade water and acetonitrile. The single component was vacuum-concentrated and submitted to structure elucidation techniques.

### 1) Structure elucidation

### 1D- and 2D NMR

NMR experiments were performed using a BRUKER Avance spectrometer (¹H 500, 13 MHz). All 1D (¹H, ¹³C) and 2D (COSY, HSQC, HMBC, NOESY) spectra were acquired in chloroform (CDCl₃). The following structure of formula (I) was identified:

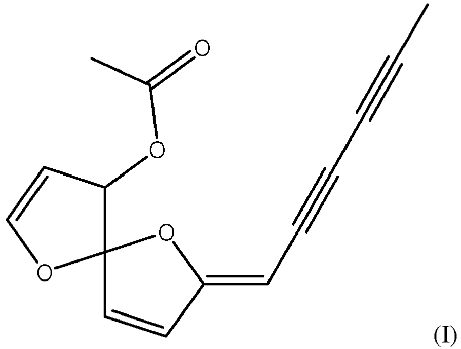

This molecule has following molecular formula: C₁₅H₁₂O₄ and following molecular weight: 256,2534 g/mol. It is already known as 1,6-dioxaspiro[4,4]nona-2,8-dien-4-ol,7-(2,4-hexadiyn-1-ylidene)-,4-acetate and belongs to the secondary metabolites polyacetylenes family.

### UPLC-HRMS

The isolated metabolite was analyzed by UPLC-ESI-HRMS using a Waters Acquity system. The inventors used UPLC-HRMS to identify the active metabolite in santolina crude extract. With the developed method, the mass-to-charge ratio (m/z) of its ion in positive mode was [M+H]⁺=257.0816 g/mol (data not shown).

This information confirmed the hypothesis of the proposed molecule, since the molecular weight of the known compound was established as M=256.2534 g/mol.

### 2) Validation of the activity of the pure compound (formula (I))

The pure compound appeared to be unstable once isolated, and particularly in aqueous and acid conditions. Since antimicrobial assays were performed in water, its antimicrobial activity could not be evaluated as for crude extracts or plant fractions. Thus, the inventors tried to establish a relationship between antimicrobial activity and active substance content in crude extracts.

### Relationship between antimicrobial activity and active substance content

A quantitation of the active substance was performed by HPLC-UV. The same solvent gradient as for HPLC-ELSD analysis was applied but stopped at 43 min. A calibration curve was determined using a trans-cinnamic acid standard (that has a retention time relatively close to the active substance retention time) prepared at different concentrations (from 0.0078 to 1 mg/mL) in methanol. Different santolina crude extracts (E1 to E2) were diluted to 20 mg/mL in methanol and analyzed by HPLC-UV at 310 nm, which is the absorption maximum of the active substance (and which allows comparison between the different extracts). The linear regression equation generated by the calibration curve permitted to calculate the value of active substance by corresponding integration and area peak measurement.

Antimicrobial activity assays (as explained in example 2) were also performed on said crude extracts, and the following results were obtained (table 4):

**Table 4: Active substance concentration and antimicrobial activity of santolina crude extracts**

| **Crude extracts** | | **Antimicrobial activity** | | | |
|---|---|---|---|---|---|
| | **Relative active substance concentration (mg/g)** | *A. niger* | *C. albicans* | *P. aeruginosa* | *S. aureus* |
| | | | | | |
| **E1** | 17,60 | + | + | + | + |
| **E2** | 0,00 | - | - | - | + |

| | | | | | |
|---|---|---|---|---|---|
| +: *strong activity,* ~: *weak activity,* -: *no activity* | | | | | |

=> The results show that a relationship between active substance concentration in crude extracts and their antimicrobial activity can be observed, especially on *A. niger* and *C. albicans.*

However, the activity on *S. aureus* seems to be independent of the presence of the active substance (formula (I)).

To confirm this hypothesis, the inventors wondered if other plants of the genus *Santolina* also containing the active substance would present the same results.

### Antimicrobial activity of other Santolina species containing the active substance

Two other *Santolina* species (*Santolina rosmarinifolia* L. and *Santolina pinnata* L.) were investigated. Crude extracts were prepared and analyzed by HPLC-ELSD to confirm the presence of the active substance. They were then submitted to the same HPLC-UV quantitation as described in the previous paragraph, and to antimicrobial activity assays, and the following results were obtained (table 5):

**Table 5 : Active substance concentration (mg of active/g of extract) and antimicrobial activity of Santolina species.**

| **Crude extracts** | **Relative active substance concentration (mg/g)** | **Antimicrobial activity** | | | |
|---|---|---|---|---|---|
| | | *A. niger* | *C. albicans* | *P. aeruginosa* | *S. aureus* |
| **S. rosmarinifolia** | 3,97 | + | + | - | + |
| **S. pinnata** | 4,56 | + | - | - | - |

| | | | | | |
|---|---|---|---|---|---|
| +: *strong activity,* -: *no activity* | | | | | |

=> The results show differences of activity between these extracts and S. *chamaecyparissus* extracts. It can be explained by differences of chemical composition of the species.

=> The presence of the active substance (formula (I)) and the antimicrobial activity can although be correlated.

### Example 5: Formulation of the extract as a natural preservative

### 1) Optimization of the extraction process

### Optimization of solvent extraction

As the inventors demonstrated that antimicrobial activity is in relation with active metabolite (compound of formula (I)) concentration, optimization of the extraction process was performed with modification of solvents.

### Ethanol/Water gradient

The initial solvent of extraction was a mix of ethanol/water 75:25 v:v. First step was also to change this gradient from 100% of ethanol to 100% of water. Seven new solvents were evaluated as described in Table 6:

**Table 6 : ETOH/Water solvent extraction gradient**

| **Ethanol (%)** | **Water (%)** | **Active substance** |
|---|---|---|
| | | **(formula (I)) concentration (mg/g)** |
| 100 (absolute EtOH) | 0 | 8.5 |
| 100 (96° EtOH) | 0 | 15.0 |
| 80 | 20 | 19.9 |
| 60 | 40 | 2.9 |
| 40 | 60 | 0.3 |
| 20 | 80 | - |
| 0 | 100 | - |

These crude extracts were then analyzed using UV-HPLC at 310 nm and the active metabolite was quantified following the method described in example 4. Higher concentration was found for EtOH/Water 80:20 v:v gradient. No active was observed in water extract, neither for weak percentage of water in extraction solvent. Higher active substance concentration was found for 80% and 96% of ethanol and decreased then for higher purity of ethanol.
=> Optimal gradient was thus between 60 and 96% v/v of ethanol.

### Other solvents

The second step was to compare other solvents to extract the plant and then quantify the metabolite concentration. Tested solvents were tetrahydrofuran, acetone, dichloromethane, methanol, absolute ethanol, ethanol 96°, ethyl acetate and methyltetrahydrofuran.

**Table 7 : Active substance concentration in function of extraction solvents**

| **Ethanol (%)** | **Active substance (formula (I)) concentration (mg/g)** |
|---|---|
| Absolute ethanol | 8.5 ± 1.2 |
| Ethanol 96° | 15.0 ± 1.0 |
| Methanol | 44.3 ± 2.6 |
| Ethyl acetate | 20.3 ± 0.8 |
| Tetrahydrofuran | 12.0 ± 0.9 |
| Acetone | 34.0 ± 0.9 |
| Dichloromethane | 40.8 ± 0.8 |
| Methyltetrahydrofuran | 16.4 ± 0.7 |

As shown in Table 7, methanol was the extraction solvent giving the higher concentration of metabolite before dichloromethane, acetone and ethyl acetate which gave very good data too.

All these 4 solvents gave higher active concentrations than ethanol/water 80:20 v/v. Methanol was also selected for the rest of the optimization of the extraction process to produce a cosmetic ingredient.

### 2) Optimization of a cosmetic preservative

Solvent extract of *Santolina chamaecyparissus* was not an easy to use ingredient for cosmetic as it was thick and doughy. To remove this drawback, a liquid and specific cosmetic raw material was added. In order to do this, extraction process was modified as follows:
After the two methanolic extractions processing of aerial parts of *Santolina chamaecyparissus* L. (see example 1), the obtained extracts were filtered and stirred together. Then, one selected liquid cosmetic raw material was added to the extract under stirring. Finally, methanol was eliminated under vacuum.

Several cosmetic raw materials were selected. They were polyols (like glycols, such as glycerol) and monoglyceride esters.

After concentration of the extract, the inventors obtained a product easier to use in cosmetic formulations.

### Extract solubility in liquid cosmetic raw material

The list of cosmetic raw materials added during the extraction process is described in Table 8.

*Santolina chamaecyparissus* was extracted (methanol extraction) according to the above protocol, and each of the cosmetic raw materials was added. Then, a classification was done in function of crude extract solubility therein as follows:

**Table 8: List of cosmetic raw materials tested for the cosmetic ingredient optimization and Santolina chamaecyparissus solubility in these products.**

| **Raw material** | ***Santolina chamaecyparissus* solubility** |
|---|---|
| Ethylhexylglycerin | Very soluble |
| Ethylhexylglycerin/Caprylyl glycol | Very soluble |
| Glyceryl caprate | Very soluble |
| Caprylyl glycol | Very soluble |
| Capryloyl glycine | Soluble |
| Glyceryl caprylate | Soluble |
| Glycerin | Moderately soluble |
| Propylene glycol | Moderately soluble |
| Butylene glycol | Moderately soluble |
| Propanediol | Moderately soluble |

Ethylhexylglycerin was the raw material in which the crude extract of *Santolina chamaecyparissus* presented the best solubility. Three of the best raw materials for the plant extract were also selected for antimicrobial activity evaluation:
- Ethylhexylglycerin,
- Ethylhexylglycerin/caprylyl glycol (Sensiva SC10 from Schülke & Mayr) and
- Caprylyl glycol.

These 3 ingredients were liquid and easy to use for cosmetic formulations.

### Metabolite concentration in optimized extract

Before biological test, HPTLC analysis were done on these three optimized extracts and compared with the crude extract without any cosmetic raw material.

### HPTLC analysis

Metabolite relative quantification was done using a Camag HPTLC system (Muttenz, Switzerland) equipped with an automatic TLC sampler (ATS4), an automatic developing chamber (ADC 2), a visualizer and a TLC scanner 4 controlled with WinCATS software. Sample solutions were applied on silica gel 60 F₂₅₄ plates (20 x 10 cm x 0,20mm) purchased from Merck. All plates were developed until 70 mm from the lower edge, with humidity control from 33 to 38 % and 20 min of saturation. Mobile phase for plates development was toluene/ethyl acetate/formic acid 98:2:1 v/v. Plates were scanned at 315 nm in reflectance mode, with D2 and W lamp, slit dimension of 8,00 mm x 0,40mm, scanning speed of 20 mm/s and data resolution of 100µm/step.

Calibration curve was performed with stock solutions of *Santolina chamaecyparissus* (crude methanolic extract) at 1 mg/mL in methanol. Several amount of the stock solution (1, 2, 6, 8 and 12 µL) were applied on plates which were then developed and scanned. The calibration plots of peak areas versus concentration were polynomial equation (second degree). R² coefficient was acceptable above 0,99. The metabolites eluted with a Rf of 0,42.

Identification of the metabolite through TLC development was performed using isolated compounds.

### HPTLC dosage

Active compound concentration was compared in each sample as described in Table after pilot extraction. A very high metabolite concentration in all products (between 31,3 and 38,9 mg of active/g of extract) was observed. Also, the addition of cosmetic raw material during the extraction process did not affect the active compound.

**Table 9: active substance concentration in Santolina chamaecyparissus extract without and with addition of cosmetic raw materials during extraction process RM1: raw material 1 (ethylhexylglycerin), RM2: raw material 2 (ethylhexylglycerin/caprylyl glycol), RM3: raw material 3 (caprylyl glycol).**

| **Ingredient** | | **Active substance concentration in the extract (mg/g)** |
|---|---|---|
| Crude extract of *Santolina chamaecyparissus* | | 38.9 ± 1.5 |
| *Santolina chamaecyparissus* RM1 | + | 31,3 ± 1.2 |
| *Santolina chamaecyparissus* RM2 | + | 36,2 ± 1.8 |
| *Santolina chamaecyparissus* RM3 | + | 33,6 ± 0.9 |

For the rest of the study, the three evaluated products are named as follows:
- "Ingredient 1": *Santolina chamaecyparissus* + Ethylhexylglycerin,
- « Ingredient 2 »: *Santolina chamaecyparissus* + Ethylhexylglycerin/caprylyl glycol and
- « Ingredient 3 *»: Santolina chamaecyparissus* + caprylyl glycol.

### Antimicrobial activity of Santolina chamaecyparissus extract with selected cosmetic

### raw materials

Antimicrobial activity of the methanolic crude extract and the three ingredients containing the crude extract and selected cosmetic raw materials was evaluated against five microorganisms at 0,4 and 0,04 % during 48 to 72 h:
- *Aspergillus niger,*
- *Escherichia coli,*
- *Candida albicans,*
- *Pseudomonas aeruginosa,* and
- *Staphylococcus aureus.*

Details of results are described in Tables 10 to 13 below.

**Table 10 : Inhibition percentage of microorganisms with crude methanolic extract of Santolina chamaecyparissus at 0,4 and 0,04 % at 24, 48 and 72 h. Inhibition > 90 % was noted +++, inhibition > 80 % and at less 70 % was noted ++, inhibition >60 % was noted +, inhibition > 60 % but lots was notes ~, Inhibition < 60 % was noted -.**

| **Sample** | **Strains** | **Inhibition percentage (%)** | | | | | | **Analyze** | |
|---|---|---|---|---|---|---|---|---|---|
| | | **24h** | | **48h** | | **72h** | | | |
| | | **0,4%** | **0,04%** | **0,4%** | **0,04%** | **0,4%** | **0,04%** | **0,4%** | **0,04%** |
| | *Aspergillus niger* | | | 93 | 50 | 91 | 12 | +++ | - |
| ***Santolina chamaecyparissus*** | *Escherichia coli* | -76 | 35 | 29 | 24 | 12 | 24 | - | - |
| Crude extract | *Candida albicans* | 103 | 2 | 41 | 17 | | | ++ | - |
| | *Pseudomonas aeruginosa* | | | 26 | 14 | 18 | 0 | - | - |
| Extraction solvent: MeOH | *Staphylococcus aureus* | 102 | -62 | 100 | -38 | | | +++ | - |

**Table 11 : Inhibition percentage of microorganisms with ingredient 1 at 0,4 and 0,04 % at 24, 48 and 72 h. Inhibition > 90 % was noted +++, inhibition > 80 % and at less 70 % was noted ++, inhibition >60 % was noted +, inhibition > 60 % but lots was notes ~, Inhibition < 60 % was noted -.**

| **Sample** | **Strains** | **Inhibition percentage (%)** | | | | | | **Analyze** | |
|---|---|---|---|---|---|---|---|---|---|
| | | **24h** | | **48h** | | **72h** | | | |
| | | **0,4%** | **0,04%** | **0,4%** | **0,04%** | **0,4%** | **0,04%** | **0,4%** | **0,04%** |
| | *Aspergillus niger* | | | 96 | 75 | 97 | 60 | +++ | + |
| ***Santolina chamaecyparissus*** | *Escherichia coli* | 100 | 39 | 101 | 54 | 98 | 61 | +++ | ~ |
| **Ingredient I** | *Candida albicans* | 107 | 3 | 101 | 10 | | | +++ | - |
| | *Pseudomonas aeruginosa* | | | 63 | 10 | 53 | 19 | ~ | - |
| Extraction solvent: MeOH | *Staphylococcus aureus* | 95 | -55 | 98 | 9 | | | +++ | - |

**Table 12 : Inhibition percentage of microorganisms with ingredient 2 at 0,4 and 0,04 % at 24, 48 and 72 h. Inhibition > 90 % was noted +++, inhibition > 80 % and at less 70 % was noted ++, inhibition >60 % was noted +, inhibition > 60 % but lots was notes ~, Inhibition < 60 % was noted -.**

| **Sample** | **Strains** | **Inhibition percentage (%)** | | | | | | **Analyze** | |
|---|---|---|---|---|---|---|---|---|---|
| | | **24h** | | **48h** | | **72h** | | | |
| | | **0,4%** | **0,04%** | **0,4%** | **0,04%** | **0,4%** | **0,04%** | **0,4%** | **0,04%** |
| | *Aspergillus niger* | | | **96** | **71** | **99** | **22** | **+++** | **~** |
| ***Santolina chamaecyparissus*** | *Escherichia coli* | 50 | 39 | 88 | 38 | 103 | 45 | ++ | - |
| **Ingredient 2** | *Candida albicans* | 104 | 14 | 85 | 13 | | | ++ | - |
| | *Pseudomonas aeruginosa* | | | 85 | -11 | 84 | 13 | ++ | - |
| Extraction solvent: MeOH | *Staphylococcus aureus* | 66 | -99 | 93 | -3 | | | + | - |

**Table 13 : Inhibition percentage of microorganisms with ingredient 3 at 0,4 and 0,04 % at 24, 48 and 72 h. Inhibition > 90 % was noted +++, inhibition > 80 % and at less 70 % was noted ++, inhibition >60 % was noted +, inhibition > 60 % but lots was notes ~, Inhibition < 60 % was noted -.**

| **Sample** | **Strains** | **Inhibition percentage (%)** | | | | | | **Analyze** | |
|---|---|---|---|---|---|---|---|---|---|
| | | **24h** | | **48h** | | **72h** | | | |
| | | **0,4%** | **0,04%** | **0,4%** | **0,04%** | **0,4%** | **0,04%** | **0,4%** | **0,04%** |
| | *Aspergillus niger* | | | 93 | 51 | 97 | 16 | +++ | - |
| ***Santolina chamaecyparissus*** | *Escherichia coli* | 114 | 32 | 98 | 28 | 97 | 36 | +++ | - |
| **Ingredient 3** | *Candida albicans* | 97 | -6 | 97 | 5 | | | +++ | - |
| | *Pseudomonas aeruginosa* | | | 103 | -26 | 101 | 8 | +++ | - |
| Extraction solvent: MeOH | *Staphylococcus aureus* | 102 | -105 | 101 | -4 | | | +++ | - |

Crude extract was active against *A. niger, C. albicans* and *S. aureus* at 0,4 %. Ingredient 1 was active against *A. niger* and *E. coli* at 0,4 and 0,04 % and against *C. albicans* and *S. aureus* at 0,4 % of *Santolina chamaecyparissus* extract. Finally, it was moderately active against *P. aeruginosa.*

Ingredient 2 was active against the five microorganisms at 0,4 % of *Santolina chamaecyparissus* extract and additionally it was active against *A. niger* at 0,04 % during 48 h.

Ingredient 3 was active against the five microorganisms at 0,4 % of *Santolina chamaecyparissus* extract.

=> Finally, a better inhibition of all ingredients was observed in comparison to the crude extract alone. These ingredients were also optimized for antimicrobial activity and cosmetic use.

### 3) Evaluation of the antimicrobial activity for cosmetic preparations

### Challenge test (description of the test)

The challenge test is designed to provide the level of biological activity possessed by the preservative system of cosmetic products. Briefly, a controlled amount of specific microorganisms is inoculated in products. Mixtures are stocked in controlled conditions and microorganisms are counted at known times for 28 days. Antimicrobial preservative effectiveness of the formulations was performed following the method recommended by the European Pharmacopeia (7^{th} edition - 2011).

### Microorganisms

Tested microorganisms were:
**Bacteria**
   *Pseudomonas aeruginosa* ATCC 9027
   *Staphylococcus aureus* ATCC 6538
   *Escherichia coli* ATCC 8739
**Fungi & mold**
   *Candida albicans* ATCC 10231
   *Aspergillus brasiliensis* ATCC 16404

These one are potential pathogenic germs and could contaminate products as they are issued from biotypes such as water, skin, intestinal or environmental.

### Culture Media

Tested strains were prepared as described in Table 14:

**Table 14 : Strain preparation**

| **Strains** | **Preservation Storage temperature** | **Culture media** | **Densities (CFU/mL)** | **Incubation temperature** |
|---|---|---|---|---|
| *P. aeruginosa* | -80°C ± 5°C | Tryptic Soy Agar (TSA) | 10⁷-10⁸ | 32,5°C ± 2,5°C |
| *S. aureus* | -80°C ± 5°C | Tryptic Soy Agar (TSA) | 10⁷-10⁸ | 32,5°C ± 2,5°C |
| *E. coli* | -80°C ± 5°C | Tryptic Soy Agar (TSA) | 10⁷-10⁸ | 32,5°C ± 2,5°C |
| *C. albicans* | -80°C ± 5°C | Sabouraud Dextrose | 10⁷-10⁸ | 22,5°C ± 2,5°C |
| *A. brasiliensis* | -80°C ± 5°C | Sabouraud Dextrose | 10⁶-10⁷ | 22,5°C ± 2,5°C |

### Procedure

The test organisms specified were to be tested separately in cosmetic products.

The product to be tested is distributed in single-use sterile flasks (20 g of product/flask) and every flask is inoculated with the suspension of one strain to be tested. Final concentration is about 10⁵ to 10⁶ microorganisms/g.

Inoculated products were to be held at 22,5 °C ± 2,5 °C in darkness during the test.

### Neutralization

Neutralization of preservative system was validated and performed on the 5 strains with LT 100 Broth at 1/10^{th} and 1/100^{th} dilution.

### Interpretation

Sampling and analysis are done after 2-day, 7-day, 14-day and 28-day period. Each sample was neutralized and enumeration method was applied (results in CFU/g). The microbial densities obtained during the follow-up for each of the tested strains are counted and compared with the thresholds of the logarithmic reduction imposed by the reference table (table 15).

**Table 15: Table of logarithmic reduction imposed by the European Pharmacopeia NI: No Increase; /: No minimal reduction required**

| **European Pharmacopeia** | | | | | |
|---|---|---|---|---|---|
| **Strains** | **Criteria** | **Logarithmic reduction** | | | |
| | | **Day-2** | **Day-7** | **Day-14** | **Day-28** |
| **Bacteria** | **A** | ≥2 | ≥3 | / | NI |
| | **B** | / | / | ≥3 | NI |
| **Fungi/mold** | **A** | / | / | ≥2 | NI |
| | **B** | / | / | ≥1 | NI |

### Cosmetic formulations

Challenge tests were realized with oil-in-water (O/W) emulsions comprising or not *Santolina chamaecyparissus* extract. The O/W emulsion formulations are described in Table 16.

**Table 16 : O/W emulsions with Santolina chamaecyparissus extract**

| | **Raw material (INCI)** | **% (w/w)** | **Procedure** |
|---|---|---|---|
| **A** | Aqua | Qsp | |
| | Glycerin | 4,00 | Heat A at 55°C |
| | | (formula 1) or 10,00 (formula 2) | |
| **B** | Acrylates/C10-30 alkyl acrylate crosspolymer | 0,15 | Add B |
| | Polyoxyethylene (2), stearyl ether | 2,00 | |
| | Steareth-21, polyethoxylated alcohol | 2,00 | |
| | Stearyl alcohol | 1,50 | |
| | Glyceryl stearate | 3,00 | |
| | Octyldodecanol | 3,00 | |
| | Squalane | 2,50 | |
| | Isohexadecane | 3,00 | Heat C at 75°C, heat A+ B at 75° and emulsified C in A+B |
| **C** | *Simmondsia chinensis* (Jojoba) seed oil | 1,50 | |
| | *Triticum vulgare* (weat) germ oil | 1,50 | |
| | Butylhydroxytoluene | 0,05 | |
| | Butyrospermum parkii butter | 2,00 | |
| | Dimethicone | 1,50 | |
| | Butyl methoxydibenzoyl-methane | 1,00 | |
| | Ethylhexyl methoxycinnamate | 1,50 | |
| | Dimethicone | 2,00 | |
| **D** | Triethanolamine | 0,11 | Add D and E at 60°C |
| **E** | Titane dioxyde, paraffin oil | 1,25 | |
| **F** | Panthenol, propylène glycol | 0,20 | |
| | Tocopheryl | 0,50 | Add F, G and H at 30°C |
| **G** | Sodium hyaluronate | 5,85 | |
| **H** | Perfume | 0,10 | |
| **I** | *Santolina chamaecyparissus* Extract | 0,5-2% | Add the preservative system at 30°C |

In the experiments, Formula 1 comprised 4% of glycerin and Formula 2 comprised 10% of glycerin.

Challenge tests were systematically performed:
- on formulations 1 and 2 comprising either Ingredients 1, 2 or 3. As Ingredients 1, 2 and 3 comprised 50% by weight of *Santolina chamaecyparissus* extract, these formulations comprised in fact 1% (for *0.5% Santolina chamaecyparissus* extract) or 4% (for 2% *Santolina chamaecyparissus* extract ) of Ingredient 1, 2 or 3, and
- on formulations 1 and 2 comprising 0.5% or 2% of ethylhexylglycerin alone (S1), of the mixture ethylhexylglycerin/caprylyl glycol alone (S2) or of caprylyl glycol alone (S3).

### Antimicrobial activity

### Antimicrobial activity in formulation 1

*Santolina chamaecyparissus* extract was first tested at 0,5 % and 2 % in formulation 1 with Ingredient 2 or 3. As ingredients 1 to 3 contained 50 % of plant extract, they were introduced at 1 and 4 % respectively.

The results are in Table 17.

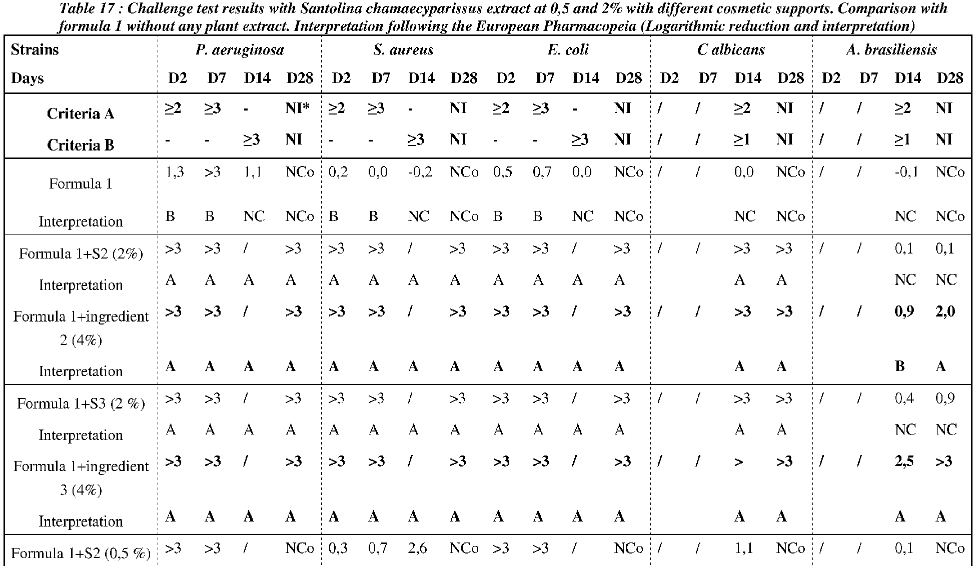

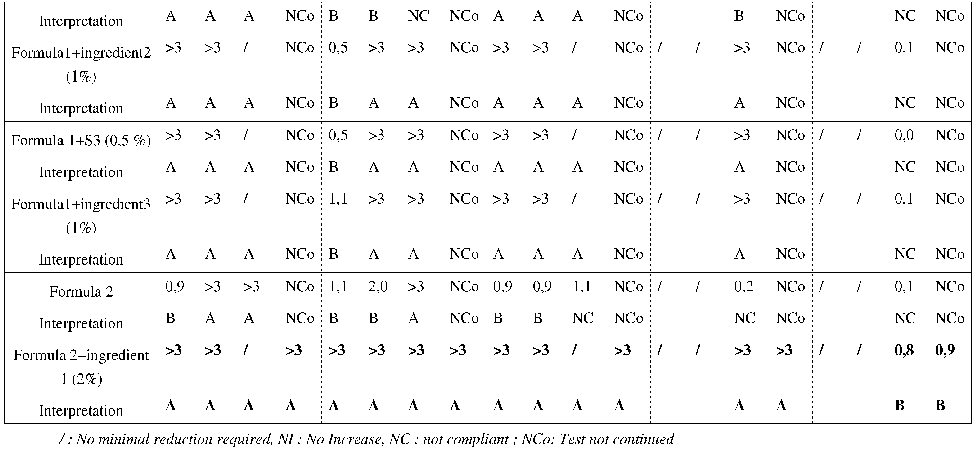

The results show that 0,5 % of *Santolina chamaecyparissus* extract was sufficient to be in accordance with criteria A of European pharmacopeia against *P. aeruginosa, E. coli* and *C. albicans* and in accordance with criteria B against *S. aureus.*

At 2% of crude extract in Ingredient 2 (= 4 % of ingredient 2), challenge test on formula 1 was in accordance with criteria A against all bacteria and yeast, and with criteria B against mold. It was noted that after 28 days of incubation, formula 1 was in keeping with criteria A against all microorganisms. The antifungal activity against *A. brasiliensis* was only slower than expected in the method.

With 2 % of *Santolina chamaecyparissus* in ingredient 3 (= 4 % of ingredient 3), challenge test on formula 1 was in accordance with criteria A against all microorganisms.

### Antimicrobial activity in formulation 2

Ingredient 1 was tested at 2 % in formulation 2 (= 1 % of crude extract). As shown in Table 17, 1 % of *Santolina chamaecyparissus* extract was sufficient to be in accordance with criteria A of European pharmacopeia against *P. aeruginosa, S. aureus, E. coli* and *C. albicans* and with criteria B against *A. brasiliensis.*

## Claims

**1.** Extract of *Santolina chamaecyparissus L.* obtained by extraction of the aerial parts with a solvent chosen from methanol, dichloromethane, ethyl acetate, acetone, tetrahydrofuran and a mixture ethanol : water in a ratio of from 70:30 % v/v to 80:20 % v/v.

**2.** Process for preparing an extract of *Santolina chamaecyparissus L*., comprising the following steps:
a) mixing aerial parts of *Santolina chamaecyparissus L.* with a solvent chosen from methanol, dichloromethane, ethyl acetate, acetone, tetrahydrofuran and a mixture ethanol : water in a ratio of from 70:30 % v/v to 80:20 % v/v,
b) macerating the mixture obtained in a) during at least 1h,
c) filtering the mixture obtained in b), so as to obtain the filtrate and the retentate, the filtrate being the extract.

**3.** Process according to claim 2, wherein the mixture ethanol : water of step a) has a ratio of from 75:25 % v/v to 80:20 % v/v.

**2.** Process according to claim 2 or 3, which comprises a further step d) comprising:
d1) mixing the retentate of step c) with the same solvent as the one used in step a),
d2) macerating the mixture obtained in d1) during at least 1h,
d3) filtering the mixture obtained in d2), so as to obtain the filtrate and the retentate, and
d4) mixing the filtrate of step c) with the filtrate of step d3), said final mixture being the extract.

**5.** Process according to any one of claims 2 to 4, which comprises a further step e) of evaporation of the solvent of the extract, such as vacuum-concentration.

**6.** Process according to claim 5, wherein step e) comprises:
e1) the addition of a cosmetic compound chosen from polyols and monoglycerides to the extract, and
e2) the evaporation of the solvent of the extract obtained in e1), such as vacuum-concentration.

**7.** Process according to claim 6, wherein the cosmetic compound of step e1) is chosen from ethylhexylglycerin, caprylyl glycol and their mixtures.

**8.** Extract of *Santolina chamaecyparissus L.* obtainable by the process according to any one of claims 2 to 7.

**9.** Extract according to claim 1 or 8, comprising the compound of formula (I):

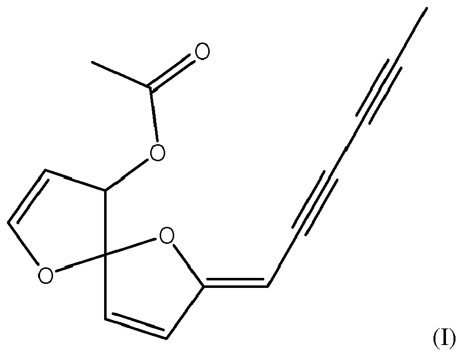

**10.** Extract according to claim 1, 8 or 9, wherein it is not an essential oil.

**11.** Composition comprising, in a physiologically acceptable medium, an extract of *Santolina chamaecyparissus L.* according to claim 1, 9 or 10, or obtainable by the process according to any one of claims 2 to 10.

**12.** Use of an extract of *Santolina chamaecyparissus L.* according to claim 1, 8, 9 or 10, or of a composition according to claim 11, as preservative, antioxidant, bactericide and/or fungicide.

**13.** Use of the compound of formula (I): 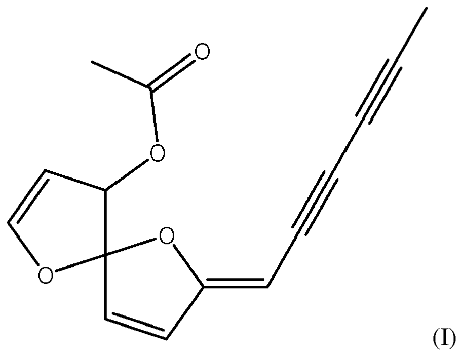 as preservative, antioxidant, bactericide and/or fungicide.

**14.** Use of a composition comprising the compound of formula (I): 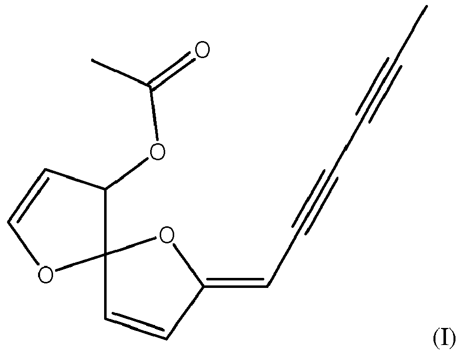 as preservative, antioxidant, bactericide and/or fungicide.
